# EUROPEAN PATENT SPECIFICATION

(11) **EP 0 567 285 B1**
(45) Date of publication and mention of the grant of the patent: **27.05.1998**
(21) Application number: 93303019.9
(22) Date of filing: 20.04.1993
(51) Int. Cl.: A61M 25/00

(54) **Surface-treated medical devices**
Oberflächenbehandelte medizinische Vorrichtungen
Dispositif médicaux traités en surface

(30) Priority: 21.04.1992 US 871644; 21.04.1992 US 871645
(43) Date of publication of application: 27.10.1993
(73) Proprietor: VANCE PRODUCTS INCORPORATED, Spencer Indiana 47460 (US)
(72) Inventor: Bosley, Rodney W., Bloomington, Indiana 47401 (US); Uldall, Peter R., Willowdale, Ontario, M2L 1M2 (CA); DeBruyne, Michael P., Bloomington, Indiana 47408 (US)
(74) Representative: Johnston, Kenneth Graham

(56) References cited:
- EP-A- 0 348 252
- EP-A- 0 386 936
- EP-A- 0 453 234
- WO-A-90/14123
- GB-A- 1 413 813

## Description

This invention relates to surface treated medical devices such as stents, catheters, cannulae, and the like.

Ultrasonic imaging in the medical field is widely used for a variety of applications. In addition to imaging physiological structures and tissue such as organs, tumors, vessels, and the like, it is often desirable for a physician or technician to have an image of a medical device which has been inserted into the tissue or passageway of a patient. The types of devices which are surgically sterilized and inserted into patients are many. Typical examples include the echogenic devices described in EP-A-0 386 936 (R.W. Bosley 4-2-2) and in copending EP-A-0 552 924 (R.W.Bosley 3-5-7).

There is a need for providing improved outer surfaces on the devices described in the above mentioned two co-pending EP Applications, and on similar devices.

EP-A-453234 describes catheter in which the outer surface of the distal end of the catheter is lubricated. In one embodiment the lubricant is in the form of a hydrophylic slip coating to provide easy insertion into an introducer sheath, and in another embodiment the lubricant is in the form of a medical grade silicone fluid spray (a non-allergenic silicone lubricant).

Other medical devices which are used for extracorporeal treatment of patients, such as hemodialysis in which blood and its components are treated outside of the body and which require access to the vascular system of the human body and, in particular, to multi-lumen catheters for use in such treatments, are described in EP-A-453,234.

A problem with catheters, stents, cannulae, and the like that are used for extracorporeal or indwelling treatment is the formation of thrombus, bacteria, fungus, and mineral encrustations when positioned in a vessel or duct for an extended period of time. This is commonly due to the rough surface of the device of which thrombus and the like readily attach thereto. In addition, the rough surface of the catheter and the like often exhibits a high coefficient of friction, which makes it difficult for inserting the device into the vessel or duct through, for example, an introducer sheath.

There is an increasing number of hemodialysis patients in whom creation and maintenance of an arteriovenous fistula is difficult or even impossible. For these patients, one prior art long-term catheter has provided a reasonable solution to their problems. It provides good blood flow and can be left in place in the external or internal jugular vein for many months or even years. The flow characteristics of this catheter are not ideal, but a plain tube catheter with an open end appears to maintain patency better than that of a tapered tube with side ports. The main problem with this plain tube catheter is its cross-sectional shape, which is similar to a double-barreled shotgun, and its squared off ends. These features make it unsuitable for percutaneous insertion over a wire guide. As a result, this plain tube catheter has to be introduced with a cut-down surgical technique, which requires considerable time and skill.

A further problem with catheters that are used for extra corporeal treatment is the formation of thrombus thereon when positioned in a blood vessel for an extended period of time. This is commonly due to the rough surface of the catheter of which the thrombus readily attach thereto. In addition, the rough surface of the catheter often exhibits a high coefficient of friction, which makes it difficult for inserting the catheter into the blood vessel through an introducer sheath.

It is an object of the present invention to improve the outer surfaces of medical devices, such as those used in ultrasonic imaging and those used in extra corporeal treatment.

EP-A-0 348 252 discloses that when the surfaces of certain polymeric materials such as polyurethane, are bombarded with certain ions, then the coefficient of friction of each of those surfaces is reduced.

According to the present invention, there is provided a surgical device as defined in claim 1.

The problems overcome by the echogenic device embodiments and the extra corporeal treatment devices of the invention, are solved and a technical advance is achieved with illustrative catheters, stents; cannulae, and the like having treated outer surfaces that are, for example, bombarded in a vacuum chamber environment with ions such as argon, nitrogen, and the like. This bombardment or treatment process smoothens the outer surfaces of the devices thereby reducing the coefficient of friction of the outer surface and making the outer surface resistant to the formation of thrombus, fungus, bacteria, and encrustations thereon.

### Brief Description of the Drawing

FIG. 1 is an enlarged view of indentations in the outer surface of a base material of an embodiment of the invention;
FIG. 2 is an enlarged view of indentations in the outer surface of a base material of an alternative embodiment of the invention with an interface layer deposited thereon; and
FIG. 3 is an enlarged cross-sectional view of another catheter of the form disclosed in EP-A-453 234.

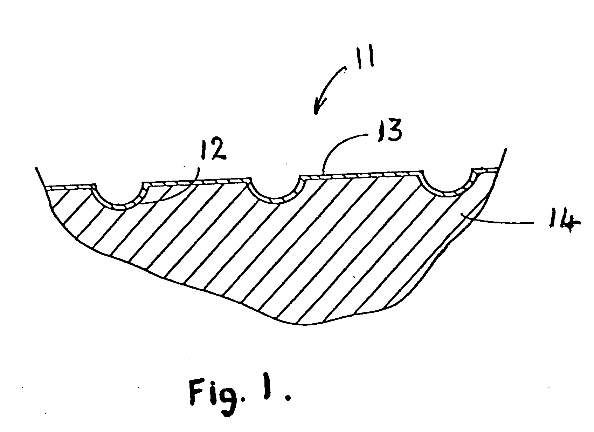

Depicted in FIG. 1 is an enlarged view of indentations 12 formed in outer surface 13 of first base material 14 of elongated member 11, such as are described in the embodiments of EP-A-0 386 936 and EP-A-0 552 924 (R.W.Bosley 3-5-7). Member 11 comprises an elongated tube having first base material 14, as previously described. Member 11 also includes outer surface 13 which has been bombarded in a vacuum chamber environment with argon ions.

In this embodiment, first base material 14 comprises a polyurethane which when untreated exhibits a first coefficient of friction of approximately 0.449. Outer surface 13 has been bombarded with ion beams and exhibits a second coefficient of friction of approximately .124. However, the elongated member material may comprise any material from a group consisting of polyurethane, polyethylene, nylon, C-FLEX™, polytetrafluoroethylene, polyvinyl chloride, polypropylene, and polyester. Alternatively, elongated member 11 comprises a silicone rubber material having a durometer in the range of 30-80 on the Shore A scale. In this particular embodiment, the silicone rubber material has a durometer of 70 on the Shore A scale. This silicone rubber material is a commercially available medical grade silicone rubber which has been treated by bombarding outer surface 13 with ions such as argon in a vacuum chamber environment. The catheter is initially formed with a first base material such as polyurethane or silicone rubber, which when untreated with argon ions exhibits a coefficient of friction of approximately .449 for untreated polyurethane and approximately .211 for untreated silicone rubber. The untreated base material catheters are placed in a vacuum chamber for argon ion beam bombardment. The untreated base material catheters are exposed to an ion argon beam while the catheter is rotated in the chamber to expose the entire outside surface of the catheter to the argon ion beam. When so treated, outer surface 13 of the base material is implanted with argon ions to smooth the surface thereof. The coefficient of friction of treated outer surface 13 is significantly reduced such as, for example, to approximately .124 for polyurethane and .125 for silicone rubber. A series of well-known fluid contact angle tests to determine the hydrophilic or hydrophobic character of the surface have indicated that a water contact angle of 75° to 95° results in a thromboresistant surface. This also results in a surface energy between 0.02 and 0.03 N/m (20 and 30 dynes per centimeter). This particular range of surface energy has been found to be resistant to the formation of blood thrombus on the surface of a device in contact with blood. It is further believed that the probable effects of ion implantation on a base material of polyurethane or silicone rubber include material densification, redistribution of surface charges, reorientation of surface chains, and chain scission followed by chain cross-linking. It is further believed that the surface of the base material is carbon enriched. It is further believed that the outer surface of polyurethane or silicone rubber with the aforementioned range of surface energy is also resistant to the formation and growth of fungus, bacteria, and encrustations. Although the above description of the catheter with treated outer surface 13 has been with respect to silicone rubber or polyurethane, any base material such as selected from the previously described group of materials bombarded with, for example, argon produces similar results. The ions used for bombardment of the outer surface varies as a function of the material selected. Other ions include the use of nitrogen, oxygen, neon, and the like for bombarding various polymer materials. Although described for use as a stent, catheter, cannulae, and the like, the outer surface treatment of any medical device intended for use in contact with any body fluid is contemplated.

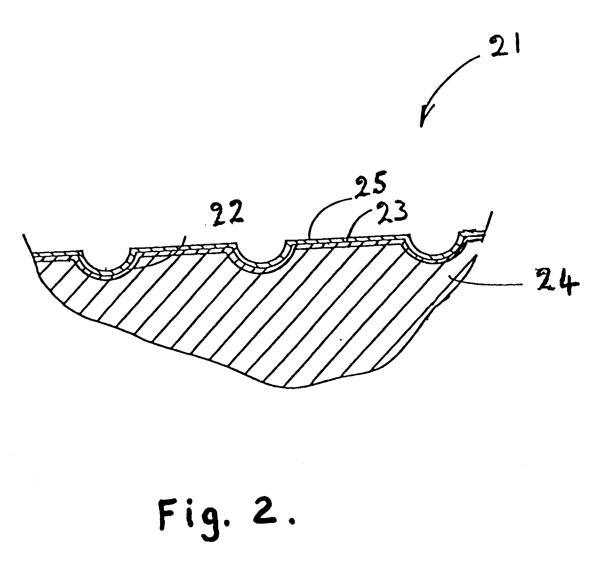

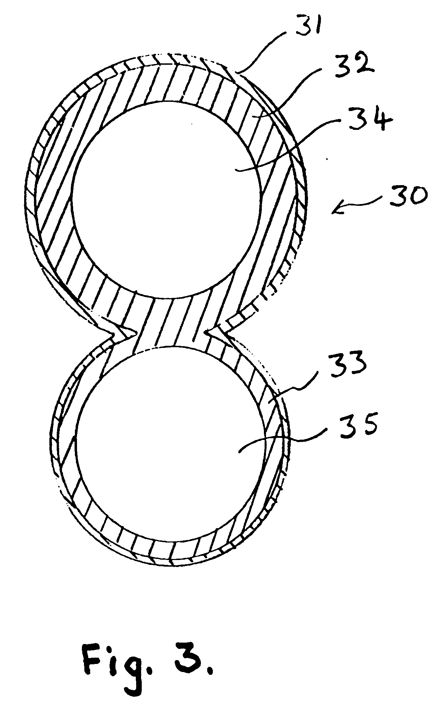

Depicted in FIG. 2 is an enlarged view of indentations 22 formed in outer surface 23 of first base material 24 of elongated member 21, which is an alternative embodiment of part or member 11 of FIG. 1. In this embodiment, an interface layer 25 has been deposited using ion beam implantation as previously described. Member 21 comprises an elongated tube having first base material 24, as previously described. Member 21 also includes outer surface 23 which has been bombarded in a vacuum chamber environment with argon ions to reduce the coefficient of friction and to bring the surface energy of the outer surface within the range of 0.02 to 0.03 N/m (20 to 30 dynes per centimeter). Interface layer includes a metal from the group consisting of titanium, tungsten, barium, bismuth, silver, gold, platinum, and palladium, which are different from base material 24, as previously described. In this embodiment with interface layer 25 deposited on outer surface 23, the coefficient of friction is approximately .184.

The ion beam treatment of the outer surface is a treatment in which the outer surface is re-oriented or altered physically as opposed to actual deposition of material by ion beam deposition techniques.

Depicted in FIG. 3 is an enlarged cross-sectional view of the distal segment of the catheter of FIG. 2 of EP-A-453,234, with outer surface 501 treated by bombardment with argon ions. In this embodiment, distal segment 30 comprises elongated members 32 and 33 with respective elongated passages 34 and 35 extending longitudinally therethrough. Elongated members 32 and 33 comprise a silicone rubber material having a durometer in the range of 30-80 on the Shore A scale. In this particular embodiment, the silicone rubber material has a durometer of 70 on the Shore A scale. Alternatively, the elongated member material may comprise any polymer material such as polyurethane, polyethylene, and the like. This silicone rubber material is a commercially available medical grade silicone rubber which has then been treated in the present embodiment by bombarding outer surface 31 with ions such as argon in a vacuum chamber environment. The catheter is initially formed with this silicone rubber material, which when untreated with argon ions exhibits a coefficient of friction of approximately .211. The untreated silicone rubber extracorporeal catheters are placed in a vacuum chamber for argon ion beam bombardment. The untreated silicone rubber is exposed to an ion argon beam while the catheter is rotated in the chamber to expose the entire outside surface of the catheter to the argon ion beam. When so treated, outer surface 31 of the silicon rubber is implanted with argon ions to smooth the surface thereof. The coefficient of friction of treated outer surface 31 is significantly reduced such as, for example, to .125. A series of well-known fluid contact angle tests to determine the hydrophilic or hydrophobic character of the surface have indicated that a water contact angle of 75° to 95° results in a thromboresistant surface. This also results in a surface energy of 0.02 to 0.03 N/m (20 to 30 dynes per centimeter). This particular range of surface energy has been found to be resistant to the formation of blood thrombus on the surface of a device in contact with blood. It is believed that the probable effects of ion implantation on silicone rubber include material densification, redistribution of surface charges, reorientation of surface chains, and chain scission followed by chain cross-linking. It is further believed that the surface of the silicone rubber is carbon enriched. It is further believed that the outer surface of silicone rubber with the aforementioned range of surface energy is also resistant to the formation and growth of fungus and bacteria. Although the above description of the distal segment 30 with treated outer surface 31 has been with respect to silicone rubber, any polymer material such as polyurethane treated with, for example, argon produces similar results. In such case, the untreated polyurethane exhibited a coefficient of friction of .449 whereas the treated polyethylene outer surface exhibited a reduced coefficient of friction of .124. The ion used for bombardment of the outer surface varies as a function of the material selected. Other ions include the use of nitrogen, oxygen, neon, and the like for bombarding vinous polymer materials. Although described for use as a hemodialysis catheter, the outer surface treatment of any catheter intended for use in contact with blood is contemplated. The internal surfaces of such catheters can be similarly treated.

A number of other grommets may be slid over or attached to the proximal segment of the catheter for anchoring the catheter to surrounding tissue. The catheter may also include any number of other connectors or clamping devices for use with the arterial and venous clamping limbs. Furthermore, the shape of the lumens may be varied to an elliptical or even crescent shape; however, the radii of lumen shapes need to be maximized to prevent or minimize turbulent blood flow and the possibility of clotting.

## Claims

1. A medical device shaped and designed for insertion, at least partially, in a passageway through which patient body fluid flows, said device comprising a part (14), of first predetermined material suitable for relatively long term insertion into the passageway, characterised in that the outer surface characteristics have been altered by bombardment with certain ions, and in that the ionic bombardment has been controlled to ensure that the said outer surface exhibits a surface energy within the range of 0.02 to 0.03 N/m (20 to 30 dynes per cm)., or a water contact angle in the range of 75 to 95°, whereby resistance of the said outer surface to the formation of thrombus, or bacteria, or fungus, or encrustation is increased.

2. A device according to claim 1, wherein said first material exhibits a first coefficient of friction when untreated, and exhibits a second lower coefficient of friction after ionic bombardment.

3. A device according to claim 1 or 2, wherein the first material comprises a polymer material, or a silicone rubber, or material selected from polyethylene, nylon, C-FLEX®, polytetrofluoroethylene, polyvinyl chloride, polypropylene, polyester, silicone.

4. A device according to claim 1,2 or 3, wherein the ionic bombardment includes bombardment by argon ions.

5. A device according to claim 1,2,3 or 4 wherein said first material includes a composite material echogenically imageable, said composite material including a solid matrix material and discrete sound reflective particles fixedly positioned throughout said matrix material ultrasonically different from tissue and more sonically reflective than said matrix material to enhance the echogenicity of said first material, said first material also including an outer layer ion beam deposited on said outer surface to further enhance the echogenicity of said elongated member.

6. A device according to claim 1,2,3, 4 or 5, wherein the said part is for insertion into the fluid passageway having a first acoustic impedance, said part including an interface layer on said first material, said interface layer having a particulate-type, discontinuous curved surface having a dimension less than a wavelength of a sonic beam from sonic imaging equipment, said interface layer including a second material different from said first material and having a second acoustic impedance different from said first acoustic impedance of said medium, said first and second acoustic impedances having at least a predetermined difference therebetween, whereby said difference between said first and second acoustic impedances and said curved surface cooperate in response to said sonic beam to produce an image of said member.

7. A device according to claim 6, wherein said particulate including a plurality of particles from a group of tungsten and glass materials in a generally spherical shape, said interface layer including a metal from a group consisting of titanium, tungsten, barium, bismuth, silver, gold, platinum and palladium different from said base material.

## Patentansprüche

1. Medizinische Vorrichtung, gestaltet und bestimmt zur mindestens teilweisen Einführung in ein von einer Körperflüssigkeit eines Patienten durchströmtes Gefäß, wobei besagte Vorrichtung aus einem Teil (14) aus einem ersten vorbestimmten, zur verhältnismäßig langdauernden Einführung in das Gefäß geeigneten Material besteht, dadurch gekennzeichnet, daß die Eigenschaften der Außenflächen durch Beschuß mit gewissen Ionen verändert worden sind und daß der Ionenbeschuß dabei so reguliert wurde, daß besagte Außenfläche eine Oberflächenenergie im Bereich von 0,02 bis 0,03 N/m (20 bis 30 Dyn pro cm) oder einen Randwinkel gegenüber Wasser im Bereich von 75 bis 95° aufweist, wodurch der Widerstand der besagten Außenfläche gegen die Bildung von Thromben oder Verkrustungen, Bakterien oder Pilzen erhöht wird.

2. Vorrichtung nach Anspruch 1, wobei besagtes erstes Material vor der Behandlung einen ersten Reibungskoeffizienten und nach dem Ionenbeschuß einen zweiten, niedrigeren Reibungskoeffizienten aufweist.

3. Vorrichtung nach Anspruch 1 oder 2, wobei das erste Material aus einem polymeren Stoff oder einem Silikonkautschuk oder einem unter Polyethylen, Nylon, C-FLEX®, Polyetetrafluorethylen, Polyvinylchlorid, Polypropylen, Polyester und Silikon ausgewählten Material besteht.

4. Vorrichtung nach Anspruch 1, 2 oder 3, wobei der Ionenbeschuß einen Beschuß mit Argonionen einschließt.

5. Vorrichtung nach Anspruch 1, 2, 3 oder 4, wobei besagtes erstes Material einen durch Echoerregung abbildbaren Verbundstoff umfaßt, wobei dieser Verbundstoff aus einer festen Grundmasse und diskreten, schallreflektierenden Teilchen besteht, die überall in der in ihrem Ultraschallverhalten von Gewebe verschiedenen Grundmasse fest angeordnet sind und eine höhere Schallreflexion als die besagte Grundmasse aufweisen, um die Echoerregbarkeit des besagten ersten Materials zu erhöhen, welches ferner auf besagter Außenfläche eine durch Ionenstrahl abgeschiedene Außenschicht aufweist, um die Echoerregbarkeit des länglichen Glieds noch weiter zu erhöhen.

6. Vorrichtung nach Anspruch 1, 2, 3, 4 oder 5, wobei der besagte Teil mit einer ersten akustischen Impedanz zur Einführung in das flüssigkeitsdurchströmte Gefäß dient und auf besagtem ersten Material eine Grenzflächenschicht aufweist, die eine partikelartige, diskontinuierliche gekrümmte Oberfläche mit kleineren Abmessungen als eine Wellenlänge eines Schallstrahls von einem Ultraschallabbildungsgerät besitzt und die ein zweites, von besagtem ersten Material verschiedenes Material mit einer zweiten, von der ersten akustischen Impedanz des besagten Mediums verschiedenen akustischen Impedanz enthält, wobei diese ersten und zweiten akustischen Impedanzen zumindest einen vorbestimmten Unterschied zwischen sich aufweisen, so daß der besagte Unterschied zwischen diesen ersten und zweiten Impedanzen und jene gekrümmte Oberfläche als Reaktion auf jenen Schallstrahl zur Erzeugung einer Abbildung des besagten Glieds zusammenwirken.

7. Vorrichtung nach Anspruch 6, wobei besagte Partikel eine Mehrzahl von Teilchen aus einer Gruppe von Wolfram- und Glasmaterialien allgemein kugelförmiger Gestalt enthalten, wobei besagte Grenzflächenschicht ein von besagtem Grundmaterial verschiedenes Metall aus einer Titan, Wolfram, Barium, Wismut, Silber, Gold, Platin und Palladium umfassenden Gruppe enthält.

## Revendications

1. Dispositif médical formé et conçu pour être inséré, au moins partiellement, dans un passage par lequel un fluide corporel du patient s'écoule, ledit dispositif comprenant une partie (14), faite d'un premier matériau prédéterminé convenant à une insertion à relativement long terme dans le passage, caractérisé en ce que les caractéristiques de la surface externe ont été altérées par bombardement avec certains ions, et en ce que le bombardement ionique a été contrôlé pour assurer que ladite surface externe présente une énergie superficielle comprise dans l'intervalle de 0,02 à 0,03 N/m (20 à 30 dynes par cm), ou un angle de contact avec l'eau dans l'intervalle de 75 à 95°, ce qui augmente la résistance de ladite surface externe à la formation de thrombus, ou aux bactéries, ou aux champignons, ou d'incrustation.

2. Dispositif suivant la revendication 1, dans lequel ledit premier matériau présente un premier coefficient de friction lorsqu'il est non traité, et présente un deuxième coefficient de friction moins élevé après le bombardement ionique.

3. Dispositif suivant la revendication 1 ou 2, dans lequel le premier matériau comprend un matériau polymère, ou un caoutchouc de silicone, ou un matériau sélectionné parmi le polyéthylène, le Nylon, le C-FLEX®, le polytétrafluoroéthylène, le poly(chlorure de vinyle), le polypropylène, le polyester, la silicone.

4. Dispositif suivant la revendication 1, 2 ou 3, dans lequel le bombardement ionique comprend le bombardement par des ions argon.

5. Dispositif suivant la revendication 1, 2, 3 ou 4, dans lequel ledit premier matériau comprend un matériau composite visualisable par échographie, ledit matériau composite comprenant un matériau de matrice solide et des particules distinctes réfléchissant les sons positionnées de manière fixe dans ledit matériau de matrice différant du point de vue des ultrasons du tissu et réfléchissant plus les sons que ledit matériau de matrice pour augmenter l'échogénicité dudit premier matériau, ledit premier matériau comprenant également une couche externe déposée par un faisceau ionique sur ladite surface externe pour encore augmenter l'échogénicité dudit membre allongé.

6. Dispositif suivant la revendication 1, 2, 3, 4 ou 5, dans lequel ladite partie est pour l'insertion dans le passage de fluide ayant une première impédance acoustique, ladite partie comprenant une couche d'interface sur ledit premier matériau, ladite couche d'interface ayant une surface à courbe discontinue, de type particulaire, ayant une dimension inférieure à une longueur d'onde d'un faisceau sonique provenant d'un appareillage de visualisation sonique, ladite couche d'interface comprenant un deuxième matériau différent dudit premier matériau et ayant une deuxième impédance acoustique différente de ladite première impédance acoustique dudit milieu, lesdites première et deuxième impédances acoustiques ayant au moins une différence prédéterminée entre elles, ladite différence entre lesdites première et deuxième impédances acoustiques et ladite surface courbée coopérant en réponse audit faisceau sonique pour produire une image dudit membre.

7. Dispositif suivant la revendication 6, dans lequel ladite matière particulaire comprend une pluralité de particules provenant d'un groupe du tungstène et de matériaux en verre de forme dans l'ensemble sphérique, ladite couche d'interface comprenant un métal, provenant d'un groupe comprenant le titane, le tungstène, le baryum, le bismuth, l'argent, l'or, le platine et le palladium, différent dudit matériau de base.
